# EUROPEAN PATENT APPLICATION

(11) **EP 4 470 555 A1**
(43) Date of publication of application: **04.12.2024**
(21) Application number: 23176254.3
(22) Date of filing: 31.05.2023
(51) Int. Cl.: A61K 39/00, C07K 14/71, C07K 14/725, C07K 16/28

(54) **ANTI-TAG CHIMERIC ANTIGEN RECEPTORS WITH SPECIFICITY FOR MUTATED PEPTIDES DERIVED FROM HUMAN FGFR2**

(71) Applicant: Miltenyi Biotec B.V. & Co. KG, 51429 Bergisch Gladbach (DE)
(72) Inventor: MITTELSTAET, Joerg, 51429 Bergisch Gladbach (DE); WEBSTER, Brian, 51429 Bergisch Gladbach (DE); ZHU, Zhongyu, 51429 Bergisch Gladbach (DE); DUDEK, Ronald P, 51429 Bergisch Gladbach (DE)
(74) Representative: Biervert, Christian

(57) **Abstract**

The present invention provides a composition comprising i) an anti-tag CAR comprising a) an antigen binding domain specific for a tag of a tagged polypeptide, b) a transmembrane domain, c) an intracellular signaling domain, and ii) said tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2, and wherein said polypeptide specifically binds to an antigen, and wherein said antigen binding domain specific for said tag binds with at least 10-fold higher affinity to said peptide than to said corresponding non-mutated peptide of said human protein FGFR2.

## Description

### Field of the invention

The present invention generally relates to the field of chimeric antigen receptors (CARs) expressed on immune cells, in particular to a combination of an Adapter CAR (anti-tag CAR) and an adapter, i.e. a tagged polypeptide, wherein said tag is a peptide derived from the human protein FGFR2.

### Background of the invention

Adoptive transfer of CAR immune cells such as CAR T cells has demonstrated remarkable success in treatment of hematological malignancies. However, lack of control of CAR immune cell function and consequent excessive inflammation in patients can result in severe side effects especially when targeting tumor-associated rather than tumor-specific antigens. Thus, temporal, tunable and spatial control of CAR activity is of major importance.

Adapter CAR immune cells such as Adapter CAR T cells are based on the dissociation of the target antigen recognition and activation domain into two complementary parts, thereby presenting a strategy to further enhance safety and flexibility of CAR immune cell systems. Anti-tag CAR immune cells do not directly recognize the tumor antigen but immune cell specificity is granted to a tag fused to an antigen-binding molecule, referred to as an adapter or adapter molecule. Such "Universal" CAR systems (or Adapter CAR (AdCAR) systems) that indirectly bind to target cells via adapters are described e.g. in WO2012082841A2, WO2013044225A1 and WO2016030414A1. The recognition of the antigen is strictly dependent on the presence of the adapter molecule giving control and the possibility for temporal on-/off-switching of CAR-mediated functions by attenuating adapter molecule administration. Furthermore, the magnitude of response can be fine-tuned by adjusting adapter concentrations. However, as functionality is dependent on the presence of the adapter molecule, patients are subjected to routine injection of the adapter molecule, which might be associated with side effects and elevated stress for the patient. Moreover, the centralized GMP-compliant production, formulation, storage, and transport of adapter molecules is time- and cost-intensive. The most relevant challenge limiting the potency of Adapter CAR immune cells, is the restricted tissue penetration and heterogenous distribution of adapter molecules in tumors upon systemic administration.

Ambrose et al developed a CAR-CD19 T cell that constitutively secretes a CD19-anti-Her2 bridging protein. This cell therapy strategy exploits the ability of CD19-targeting CAR T cells to interact with CD19 on normal B cells to drive expansion, persistence and fitness. The secreted bridging protein potently binds to Her2-positive tumor cells, mediating CAR-CD19 T cell cytotoxicity *in vitro* and *in vivo* (Doi: 10.1101/2020.03.25.007658).

WO2017075537A1 discloses a cell comprising a constitutive expression construct encoding a fusion protein comprising (a) an antigen-binding protein or fragment that binds a tumor antigen; and (b) a polypeptide target for a cellular therapeutic, antibody, or antibody-drug conjugate. WO2018156802A1 discloses a cell comprising a constitutive expression construct encoding a fusion protein comprising (a) an antigen-binding protein or fragment that binds a tumor antigen; and (b) an anti-idiotype antibody or fragment, or an anti-idiotype peptide, that binds an antigen binding domain of a cellular therapeutic, antibody, or antibody-drug conjugate. WO2016030414A1 discloses a nucleic acid encoding a universal chimeric antigen receptor, wherein the receptor comprises three domains, wherein the first domain is a tag-binding domain, the second domain is an extracellular hinge and a transmembrane domain and the third domain is a signal transduction domain, wherein the tag-binding domain binds to a tag derived from any human nuclear protein.

WO2022049217A1 discloses the use of inducible adapters, i.e. they are inducible in immune cells, such as the His₆-tagged anti-CD19-Fab for use in combination of an Adapter CAR (anti-tag CAR).

There is a need in the art for an improved or alternative Adapter CAR system comprising Adapter CARs and adapters.

### Brief description of the invention

Surprisingly it was found that an adapter (a tagged polypeptide) as disclosed herein for an Adapter CAR (an anti-tag CAR; AdCAR) has a high cytotoxicity against target cells, i.e. show durable tumor clearance in in-vivo mice studies and simultaneously do not show observable toxicity in the mice, i.e. they show minimal or no side effects during an immunotherapy in a subj ect.

The Adapter CAR system as disclosed herein may comprise
i) an Adapter CAR (an anti-tag CAR) comprising
   a) an antigen binding domain specific for a tag of a tagged polypeptide (an adapter)
   b) a transmembrane domain, and
   c) an intracellular signaling domain, and
ii) said tagged polypeptide, wherein said tag of said tagged polypeptide may be a peptide of the human protein FGFR2, and wherein the amino acid sequence of said peptide may bear at least one mutation as compared to (relative to) the corresponding non-mutated peptide (Wild type) of said human protein, and wherein said tagged polypeptide specifically may bind to an antigen, and wherein said antigen binding domain specific for said tag may bind with at least 10-fold, at least 15-fold, at least 20-fold, at least 50-fold or at least 100-fold lower affinity to the corresponding non-mutated peptide (wild type) of said human protein FGFR2 relative to the binding to said tag.

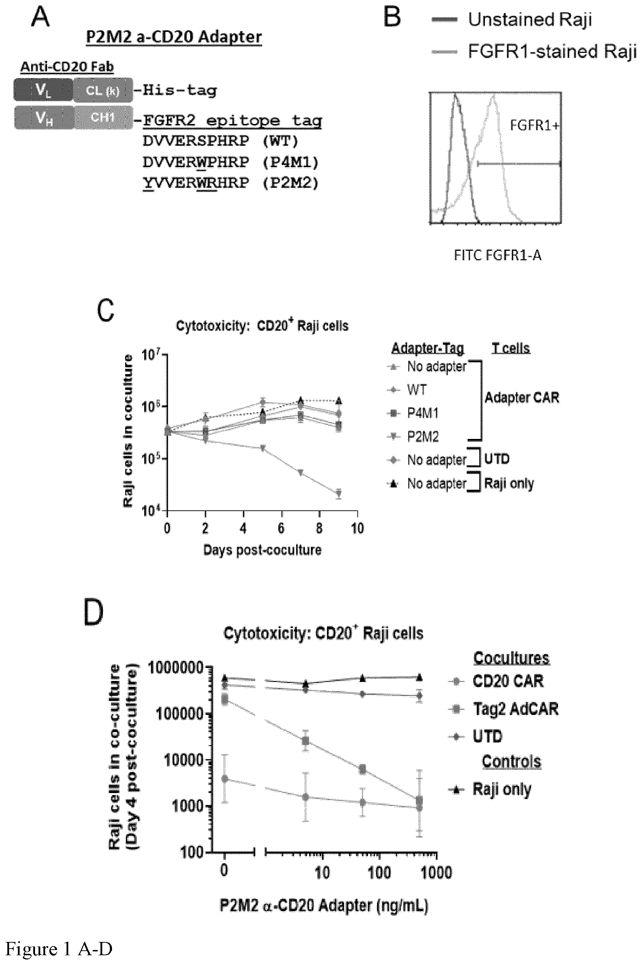

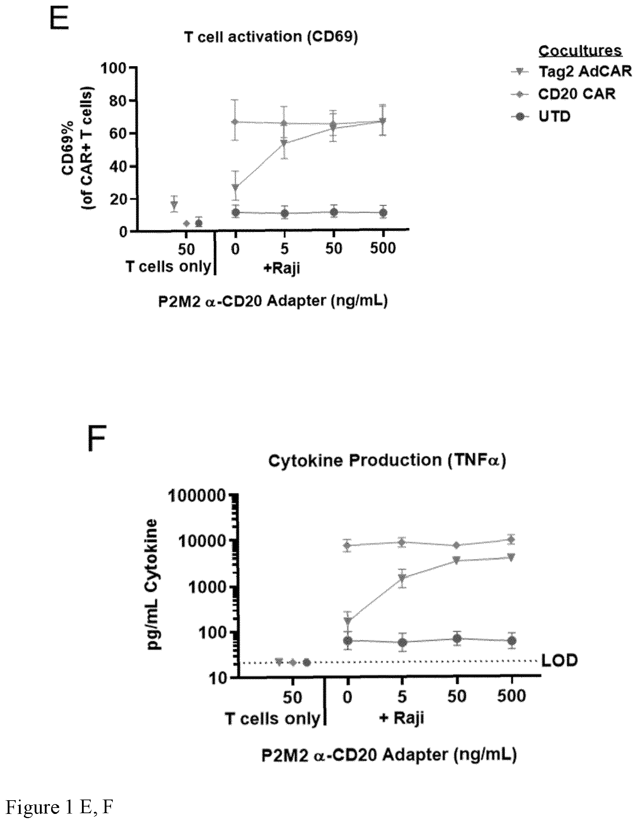

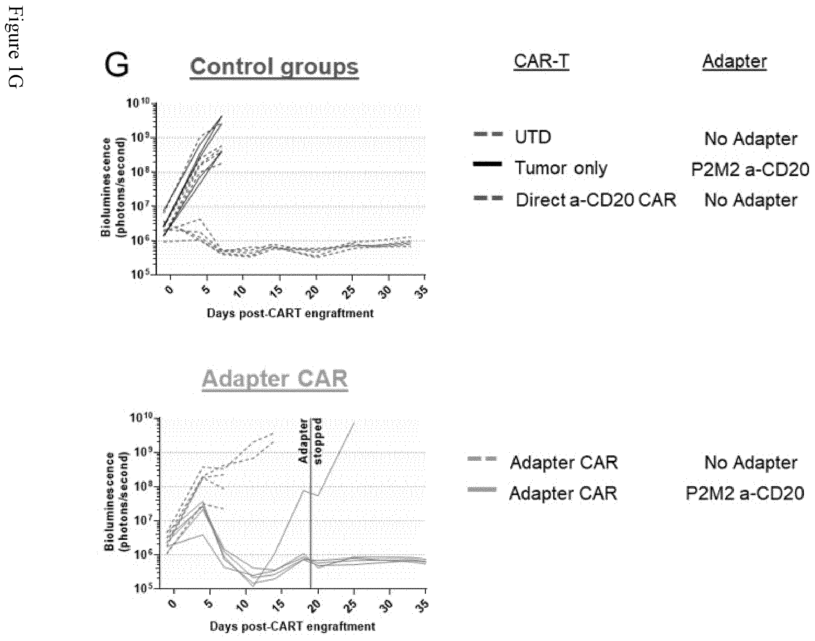

Exemplary the Adapter CAR system as disclosed herein is shown with tags derived from the human FGFR2 protein and antigen binding domains of the CAR that are specific for these tags. It is disclosed herein that a peptide which carries one mutation away from the wild type peptide sequence ("wt peptide" having SEQ ID NO:1) of the human FGFR2 protein having SEQ ID NO:2 (the peptide is referred to herein as "P4M1") and its associated antigen binding domain in the Adapter CAR having SEQ ID NO:4 (the antigen binding domain is referred to herein as "anti-P4M1") displays beneficial characteristics (see Figure 1). Even more beneficial characteristics shows the pair P2M2/anti-P2M2, wherein P2M2 is a peptide which carries three mutations away from the wild type peptide sequence of the human FGFR2 protein having SEQ ID NO:3 and anti-P2M2 is its associated antigen binding domain within the Adapter CAR having SEQ ID NO:5. The Adapter CAR system using P2M2/anti-P2M2 shows durable tumor clearance in in-vivo mice studies (Figure 1).

But no restriction of the invention to these examples of peptides of the human FGRF2 is intended as the concept can be transferred to other peptides (tags) of the human protein FGFR2 that carry other mutations as disclosed herein.

### Brief description of the drawings

Figure 1A-G. Use of anti-P2M2 FGFR2 Adapter CARs against CD20+ Raji NHL tumor cells.
Figure 2A-F. Use of anti-P2M2 FGFR2 Adapter CARs against CD33+ OCI-AML2 tumor cells.
Figure 3A-C. Use of in situ-produced adapter with anti-P2M2 FGFR2 Adapter CARs

### Detailed description of the invention

In a first aspect the present invention provides a composition (or a combination or a system) comprising
i) an anti-tag CAR comprising
   a) an antigen binding domain specific for a tag of a tagged polypeptide
   b) a transmembrane domain
   c) an intracellular signaling domain, and
ii) said tagged polypeptide, wherein said tag of said tagged polypeptide may be a peptide of the human protein FGFR2, and wherein the amino acid sequence of said peptide may bear at least one mutation as compared to the corresponding non-mutated peptide (Wild type) of said human protein FGFR2,
   and wherein said polypeptide may specifically bind to an antigen,
   and wherein said antigen binding domain specific for said tag may bind with at least 10-fold, at least 15-fold, at least 20-fold, at least 50-fold or at least 100-fold higher affinity to said peptide (tag) than to said corresponding non-mutated peptide of said human protein FGFR2.

Said intracellular (cytoplasmic) signaling domain may comprise at least one primary cytoplasmic signaling domain comprising an immunoreceptor tyrosine-based activation motif (ITAM) and/or at least one co-stimulatory signaling domain.

Said primary cytoplasmic signaling domain of said first CAR may be CD3zeta.

Said at least one co-stimulatory domain of said first CAR, may be selected from the group consisting of ICOS, CD154, CD5, CD2, CD46, HVEM, CD8, CD97, TNFRSF18, CD30, SLAM, DAP10, CD64, CD16, CD89, MyD88, KIR-2DS, KIR-3DS, NKp30, NKp44, NKp46, NKG2D, ICAM, CD27, OX40, 4-1BB, and CD28.

Said an antigen binding domain specific for a tag of a tagged polypeptide may be an antibody or antigen binding fragment thereof such as a Fab, a scFv or a nanobody.

Said polypeptide that may specifically bind to an antigen may be an antibody or antigen binding fragment thereof such as a Fab, a scFv or a nanobody.

Said antigen may be an antigen expressed on the surface of a target cell.

Said antigen may be a tumor associated antigen (TAA). Said target cell may be a tumor cell.

Said antigen may be expressed by a cell of the tumor microenvironment. Said antigen may be a soluble antigen expressed by a cell of the tumor microenvironment.

Said antigen may be an infectious pathogen-associated antigen (e.g., from human immunodeficiency virus or other viruses). Said target cell may be a pathogen-infected cell.

Said antigen may be antigen specifically associated with an autoimmune disease.

Said antigen may be antigen specifically associated with an allergic disease.

Said antigen may be selected from the group consisting of CD33 (Siglec-3), CD123 (IL3RA), CD135 (FLT-3), CD44 (HCAM), CD44V6, CD47, CD184 (CXCR4), CLEC12A (CLL1), LeY, FRβ, MICA/B, CD305 (LAIR-1), CD366 (TIM-3), CD96 (TACTILE), CD133, CD56, CD29 (ITGB1), CD44 (HCAM), CD47 (IAP), CD66 (CEA), CD112 (Nectin2), CD117 (c-Kit), CD133, CD146 (MCAM), CD155 (PVR), CD171 (L1CAM), CD221 (IGF1), CD227 (MUC1), CD243 (MRD1), CD246 (ALK), CD271 (LNGFR), CD19, CD20, GD2, EGFR, EGFRvIII, Her2, CD19 and CD276.

Said tag (i.e. said peptide) of said tagged polypeptide may comprise between 8 to 30 amino acids, between 8 to 20 amino acids, between 8 to 15 amino acids, between 9 to 11 amino acids or may have about 10 amino acids or may have 10 amino acids.

Said composition, wherein said tagged polypeptide may be a fusion protein comprising or consisting of said tag, i.e. said peptide, and said polypeptide specific for an antigen. In specific embodiments of the invention the tagged polypeptide may comprise an additional amino acid sequence between said tag and said polypeptide. One example of such additional amino acid sequence may be a G₄S sequence.

Said composition, wherein said at least one mutation may be a mutation that is commonly mutated (or prevalent) in said human protein FGFR2 in cancer.

Said composition, wherein said peptide (i.e. said tag) comprises two or more mutations as compared to the corresponding non-mutated peptide (Wild type) of said human protein FGFR2, and wherein in each of said mutations is commonly found in cancer.

Said composition, wherein said peptide of said human protein comprises or consists of SEQ ID NO:3 (P2M2) and wherein said antigen binding domain specific for a tag comprises SEQ ID NO:5 (anti-P2M2), or wherein said peptide of said human protein comprises or consists of SEQ ID No:2 (P4M1) and wherein said antigen binding domain specific for a tag comprises SEQ ID NO:4 (anti-P4M1).

In another aspect the present invention provides a composition (or combination or system) comprising
i) an immune cell expressing an anti-tag CAR comprising
   a) an antigen binding domain specific for a tag of a tagged polypeptide
   b) a transmembrane domain
   c) an intracellular signaling domain, and
ii) said tagged polypeptide, wherein said tag of said tagged polypeptide is a peptide of the human protein FGFR2, and wherein the amino acid sequence of said peptide bears at least one mutation as compared to the corresponding non-mutated peptide (Wild type) of said human protein FGRF2,
   and wherein said polypeptide specifically binds to an antigen,
   and wherein said antigen binding domain specific for said tag binds with at least 10-fold, at least 15-fold, at least 20-fold, at least 50-fold or at least 100-fold higher affinity to said peptide (tag) than to said corresponding non-mutated peptide of said human protein FGFR2.

In a further aspect the present invention provides a composition (or combination or a system or a kit) comprising
i) a (isolated) nucleic acid sequence comprising encoding an anti-tag CAR comprising
   a) an antigen binding domain specific for a tag of a tagged polypeptide
   b) a transmembrane domain
   c) an intracellular signaling domain, and
ii) a (isolated) nucleic acid sequence comprising encoding said tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2,
   and wherein said polypeptide specifically binds to an antigen,
   and wherein said antigen binding domain specific for said tag binds with at least 10-fold, at least 15-fold, at least 20-fold, at least 50-fold or at least 100-fold higher affinity to said peptide (tag) than to said corresponding non-mutated peptide of said human protein FGFR2.

Said composition, wherein said peptide of said human protein comprises or consists of SEQ ID NO:3 (P2M2) and wherein said antigen binding domain specific for a tag comprises SEQ ID NO:5 (anti-P2M2), or wherein said peptide of said human protein comprises or consists of SEQ ID No:2 (P4M1) and wherein said antigen binding domain specific for a tag comprises SEQ ID NO:4 (anti-P4M1).

In another aspect the present invention provides an immune cell comprising
i) a nucleic acid sequence comprising encoding an anti-tag CAR comprising
   a) an antigen binding domain specific for a tag of a tagged polypeptide
   b) a transmembrane domain
   c) an intracellular signaling domain, and
ii) a nucleic acid sequence comprising encoding said tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2,
   and wherein said polypeptide specifically binds to an antigen,
   and wherein said antigen binding domain specific for said tag binds with at least 10-fold, at least 15-fold, at least 20-fold, at least 50-fold or at least 100-fold higher affinity to said peptide (tag) than to said corresponding non-mutated peptide of said human protein FGFR2.

Said immune cell, wherein said immune cell additionally comprises an inducible gene expression system comprising
I) a first nucleic acid sequence comprising an inducible promoter operably linked to a second nucleic acid sequence that is said nucleic acid sequence comprising encoding said tagged polypeptide
II) a third nucleic acid sequence that is said nucleic acid sequence comprising encoding an anti-tag CAR,
and wherein said inducible gene expression system is an antigen-activated inducible gene expression system, and said antigen-activated inducible promoter is an antigen-activated promoter capable of driving expression of said tagged polypeptide when said immune cell is activated by said antigen.

Said immune cell, wherein said nucleic acid sequence comprising encoding said anti-tag CAR may comprise a constitutive promoter leading to a constitutive expression of said anti-tag CAR in said immune cell.

Said immune cell, wherein said immune cell may be a tumor infiltrating lymphocyte (TIL), a T cell or an NK cell.

In a further aspect the invention provides a composition comprising
a) a first immune cell comprising an inducible gene expression system comprising
   a first nucleic acid sequence comprising an inducible promoter operably linked to a second nucleic acid sequence (comprising) encoding a tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2, and wherein said polypeptide specifically binds to an antigen,
b) a second immune cell comprising a third nucleic acid sequence encoding an anti-tag CAR comprising
   i) an antigen binding domain specific for a tag of a tagged polypeptide
   ii) a transmembrane domain
   iii) an intracellular signaling domain, and
      and wherein said antigen binding domain specific for said tag binds with at least 10-fold, at least 15-fold, at least 20-fold, at least 50-fold or at least 100-fold higher affinity to said peptide (tag) than to said corresponding non-mutated peptide of said human protein FGFR2,
      and wherein said inducible gene expression system is an antigen-activated inducible gene expression system, and said antigen-activated inducible promoter is an antigen-activated promoter capable of driving expression of said tagged polypeptide when said first immune cell is activated by said antigen.

Said composition, wherein said first immune cell is a TIL, a T cell or an NK cell, and wherein said second immune cell is a TIL, a T cell or NK cell.

Said composition, wherein said first immune cell is a TIL, and wherein said second immune cell is a TIL, a T cell or NK cell.

Said composition, wherein said first immune cell is a TIL, and wherein said second immune cell is a T cell or NK cell.

In a further aspect the invention provides a composition comprising
a) a first immune cell comprising an inducible gene expression system comprising a first nucleic acid sequence comprising an inducible promoter operably linked to a second nucleic acid sequence encoding a tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2, and wherein said polypeptide specifically binds to an antigen,
b) a second immune cell comprising a third nucleic acid sequence encoding an anti-tag CAR comprising
   i) an antigen binding domain specific for a tag of a tagged polypeptide
   ii) a transmembrane domain
   iii) an intracellular signaling domain, and
      and wherein said antigen binding domain specific for said tag binds with at least 10-fold higher affinity to said peptide than to said corresponding non-mutated peptide of said human protein FGFR2,
      and wherein said inducible gene expression system is a drug-inducible expression system and said inducible promoter is a drug-inducible promoter, wherein said inducible gene expression system further comprises a nucleic acid encoding a synthetic transcription factor for said drug-inducible promoter, wherein when a drug is administered to a cell having said inducible gene expression system, the gene expression system is induced and the tagged polypeptide is expressed.

Said composition, wherein said synthetic transcription factor comprises a DNA binding domain and drug-binding domain and an activation domain, wherein said synthetic transcription factor is activated by binding to said drug.

The term "synthetic transcription factor" as used herein may comprise a DNA-binding domain, a drug inducible domain (a drug binding domain) and an effector (activation) domain, that are linked and/or fused whereby the individual domains can be arranged in any order.

A DNA binding domain of a synthetic transcription factor may be a protein or a portion of a protein that specifically recognize the DNA binding motif of the drug-inducible promoter and mediate the binding of the synthetic transcription factor to this DNA sequence. Besides zinc finger proteins, TALE (transcription activator-like effector) and Cas9 (Clustered Regulatory Interspaced Short Palindromic Repeats -associated system) may be engineered to recognize a specific DNA sequence. Moreover, the DNA binding domain of naturally occurring transcription factors (e.g. POU homeodomain) may be employed.

Said DNA binding domain may be e.g. a zinc finger protein (or the DNA binding domain thereof) or a protein comprising or consisting of a POU domain.

DNA binding motifs of drug-inducible promoters are specific DNA sequences that are directly or indirectly (in case of Cas9) recognized by the DNA-binding domain of the synthetic transcription factor. E.g. each zinc finger domain specifically recognizes a DNA sequence of 3 bp, thus a three-finger zinc finger protein can be designed to recognize a 9 bp sequence.

Drug-binding domain of a synthetic transcription factor refers to a protein or a portion of a protein that binds to a drug or a ligand of the domain. Upon drug binding, the drug-binding domain enables the transition from an inactive to an active synthetic transcription factor. This transition may include the release of inactivation factors and/or the translocation of the synthetic transcription factor from the cytoplasm to the nucleus. Examples of drug binding domains are nuclear receptors, extracellular domains of receptors, antigen/substance binding proteins (also dimerizers) and/or active sites of enzymes.

An activation domain of a synthetic transcription factor refers to a protein or a portion of a protein that autonomously facilitates the recruitment of the transcriptional machinery to initiate mRNA transcription. Examples of activation domains are VP16, VP64, fragments ofNFkB p65, heat shock factor 1 and combinations thereof.

E.g. the synthetic transcription factor may comprise a zinc finger protein, the estrogen receptor (ER) and an activation domain, and wherein said drug may be tamoxifen or a tamoxifen metabolite. Said activation domain may be e.g. herpes virus simplex protein VP16, the tetrameric repeat of VP16's minimal activation domain VP64, parts of the p65 domain of the human endogenous transcription factor NFκB or a fusion protein comprising fragments of human NFκB p65 and heat shock factor 1. Said tamoxifen metabolite may be endoxifen or 4-OHT. Said ER may be a ER having point mutations such as murine ER (G525R) or (G521R), human ER (G400V, M543A, L540A) or human ER (G400V, M543A, L544A).

The drug-inducible promoter may be a hybrid promoter comprising a DNA binding motif for said DNA binding domain of the synthetic transcription factor and a minimal promoter.

Said drug-inducible promoter may be a hybrid promoter comprising a zinc finger binding motif and a minimal promoter that comprises a minimal promoter selected from the group consisting of E1b, TK, IL2, CMV, SV40.

In one aspect the present invention provides a composition comprising
i) an anti-tag CAR comprising
   a) an antigen binding domain comprising or consisting of SEQ ID NO:5 (anti-P2M2).
   b) a transmembrane domain
   c) an intracellular signaling domain, and
ii) a tagged polypeptide, wherein said tag of said tagged polypeptide comprises or consists of SEQ ID NO:3 (P2M2), and wherein said polypeptide specifically binds to an antigen.

In another aspect the present invention provides a composition comprising
i) an anti-tag CAR comprising
   a) an antigen binding domain comprising or consisting of SEQ ID NO:4 (anti-P4M1).
   b) a transmembrane domain
   c) an intracellular signaling domain, and
ii) a tagged polypeptide, wherein said tag of said tagged polypeptide comprises or consists of SEQ ID NO:2 (P4M1), and wherein said polypeptide specifically binds to an antigen.

In one aspect the present invention provides a composition (or kit) comprising
i) a (isolated) nucleic acid sequence (comprising) encoding an anti-tag CAR comprising
   a) an antigen binding domain comprising or consisting of SEQ ID NO:5 (anti-P2M2).
   b) a transmembrane domain
   c) an intracellular signaling domain, and
ii) a (isolated) nucleic acid sequence (comprising) encoding a tagged polypeptide, wherein said tag of said tagged polypeptide comprises or consists of SEQ ID NO:3 (P2M2), and wherein said polypeptide specifically binds to an antigen.

In another aspect the present invention provides a composition (or a kit) comprising
i) a (isolated) nucleic acid sequence (comprising) encoding an anti-tag CAR comprising
   a) an antigen binding domain comprising or consisting of SEQ ID NO:4 (anti-P4M1).
   b) a transmembrane domain
   c) an intracellular signaling domain, and
ii) a (isolated) nucleic acid sequence (comprising) encoding a tagged polypeptide, wherein said tag of said tagged polypeptide comprises or consists of SEQ ID NO:2 (P4M1), and wherein said polypeptide specifically binds to an antigen.

In a further aspect the present invention provides an in-vitro method for generation of anti-tag CARs comprising
a) providing
   i) a peptide of the human protein FGFR2, wherein the amino acid sequence of said peptide bears at least one mutation as compared to the corresponding non-mutated peptide (wt) of said human protein FGFR2,
   ii) said non-mutated peptide (wt) of said human protein FGFR2, and
   iii) a human scFv library
b) subtractively removing (subractively screening) scFvs from said human scFv library binding against said non-mutated peptide, thereby removing scFvs binding under screening conditions to the non-mutated peptide from said human scFv library
c) screening said human scFv library of step b) for scFv's against said peptide
d) substractively removing (substractively screening) scFv's of step c) that do not have an at least 5-fold higher affinity for said peptide as compared to said non-mutated peptide
e) identifying from said scFv's of step d) that bind with an affinity of at least 1uM to said peptide
f) incorporation of one of said scFv's of step e) as an antigen binding domain of a CAR that comprises the structure:
   I) said antigen binding domain
   II) a transmembrane domain
   II) an intracellular signaling domain,
      thereby generating an anti-tag CAR that is specific for said peptide.

In another aspect the present invention provides a combination of compositions for use in treatment of a disease such as cancer, autoimmune disease or infectious disease, comprising
i) an immune cell expressing an anti-tag CAR comprising
   a) an antigen binding domain specific for a tag of a tagged polypeptide
   b) a transmembrane domain
   c) an intracellular signaling domain, and
ii) said tagged polypeptide, wherein said tag of said tagged polypeptide is a peptide of the human protein FGFR2, and wherein the amino acid sequence of said peptide bears at least one mutation as compared to the corresponding non-mutated peptide (Wild type) of said human protein FGFR2,
   and wherein said polypeptide specifically binds to an antigen,
   and wherein said antigen binding domain specific for said tag binds with at least 10-fold, at least 15-fold, at least 20-fold, at least 50-fold or at least 100-fold higher affinity to said peptide (tag) than to said corresponding non-mutated peptide of said human protein FGFR2.

In another aspect the present invention provides a composition comprising an immune cell for use in treatment of a disease such as cancer, autoimmune disease or infectious disease, the immune cell comprising
i) a nucleic acid sequence (comprising) encoding an anti-tag CAR comprising
   a) an antigen binding domain specific for a tag of a tagged polypeptide
   b) a transmembrane domain
   c) an intracellular signaling domain, and
ii) a nucleic acid sequence (comprising) encoding said tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2,
   and wherein said polypeptide specifically binds to an antigen,
   and wherein said antigen binding domain specific for said tag binds with at least 10-fold, at least 15-fold, at least 20-fold, at least 50-fold or at least 100-fold higher affinity to said peptide (tag) than to said corresponding non-mutated peptide of said human protein FGFR2.

In another aspect the present invention provides a composition of immune cells for use in treatment of a disease such as cancer, autoimmune disease or infectious disease, the immune the composition comprising
a) a first immune cell comprising an inducible gene expression system comprising
   a first nucleic acid sequence comprising an inducible promoter operably linked to a second nucleic acid sequence (comprising) encoding a tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2, and wherein said polypeptide specifically binds to an antigen,
b) a second immune cell comprising a third nucleic acid sequence encoding an anti-tag CAR comprising
   i) an antigen binding domain specific for a tag of a tagged polypeptide
   ii) a transmembrane domain
   iii) an intracellular signaling domain, and
      and wherein said antigen binding domain specific for said tag binds with at least 10-fold, at least 15-fold, at least 20-fold, at least 50-fold or at least 100-fold higher affinity to said peptide (tag) than to said corresponding non-mutated peptide of said human protein FGFR2,
      and wherein said inducible gene expression system is an antigen-activated inducible gene expression system, and said antigen-activated inducible promoter is an antigen-activated promoter capable of driving expression of said tagged polypeptide when said first immune cell is activated by said antigen.

In another aspect the present invention provides a pharmaceutical composition comprising an immune cell comprising
A) an immune cell comprising
   i) a nucleic acid sequence (comprising) encoding an anti-tag CAR comprising
      a) an antigen binding domain specific for a tag of a tagged polypeptide
      b) a transmembrane domain
      c) an intracellular signaling domain, and
   ii) a nucleic acid sequence (comprising) encoding said tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2,
      and wherein said polypeptide specifically binds to an antigen,
      and wherein said antigen binding domain specific for said tag binds with at least 10-fold, at least 15-fold, at least 20-fold, at least 50-fold or at least 100-fold higher affinity to said peptide (tag) than to said corresponding non-mutated peptide of said human protein FGFR2, and optionally
B) a pharmaceutically acceptable carrier.

Pharmaceutically acceptable carriers, diluents or excipients may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

In another aspect the invention provides a pharmaceutical composition comprising immune cells comprising
a) a first immune cell comprising an inducible gene expression system comprising
   a first nucleic acid sequence comprising an inducible promoter operably linked to a second nucleic acid sequence (comprising) encoding a tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2, and wherein said polypeptide specifically binds to an antigen,
b) a second immune cell comprising a third nucleic acid sequence encoding an anti-tag CAR comprising
   i) an antigen binding domain specific for a tag of a tagged polypeptide
   ii) a transmembrane domain
   iii) an intracellular signaling domain, and
      and wherein said antigen binding domain specific for said tag binds with at least 10-fold, at least 15-fold, at least 20-fold, at least 50-fold or at least 100-fold higher affinity to said peptide (tag) than to said corresponding non-mutated peptide of said human protein FGFR2,
      and wherein said inducible gene expression system is an antigen-activated inducible gene expression system, and said antigen-activated inducible promoter is an antigen-activated promoter capable of driving expression of said tagged polypeptide when said first immune cell is activated by said antigen, and optionally
c) a pharmaceutically acceptable carrier.

In a further aspect the invention provides an *in-vivo* method for treating a subject suffering from a disease comprising
administering to said subject an immune cell comprising
i) a nucleic acid sequence (comprising) encoding an anti-tag CAR comprising
   a) an antigen binding domain specific for a tag of a tagged polypeptide
   b) a transmembrane domain
   c) an intracellular signaling domain, and
ii) a nucleic acid sequence (comprising) encoding said tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2,
   and wherein said polypeptide specifically binds to an antigen,
   and wherein said antigen binding domain specific for said tag binds with at least 10-fold, at least 15-fold, at least 20-fold, at least 50-fold or at least 100-fold higher affinity to said peptide (tag) than affinity to said corresponding non-mutated peptide of said human protein FGFR2. Said disease may be cancer, an autoimmune disease or an infectious disease.

In a further aspect the invention provides an *in-vivo* method for treating a subject suffering from a disease comprising
I) administering to said subject an immune cell comprising
   i) a nucleic acid sequence (comprising) encoding an anti-tag CAR comprising
      a) an antigen binding domain specific for a tag of a tagged polypeptide
      b) a transmembrane domain
      c) an intracellular signaling domain,
   ii) administering to said subject said tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2, and wherein said polypeptide specifically binds to an antigen, and wherein said antigen binding domain specific for said tag binds with at least 10-fold, at least 15-fold, at least 20-fold, at least 50-fold or at least 100-fold higher affinity to said peptide (tag) than to said corresponding non-mutated peptide of said human protein FGFR2.

In a further aspect the present invention provides an *in-vivo* method for treating a subject suffering from a disease comprising administering to said subject
a) a first immune cell comprising an inducible gene expression system comprising a first nucleic acid sequence comprising an inducible promoter operably linked to a second nucleic acid sequence (comprising) encoding a tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2, and wherein said polypeptide specifically binds to an antigen,
b) a second immune cell comprising a third nucleic acid sequence encoding an anti-tag CAR comprising
   i) an antigen binding domain specific for a tag of a tagged polypeptide
   ii) a transmembrane domain
   iii) an intracellular signaling domain, and
      and wherein said antigen binding domain specific for said tag binds with at least 10-fold, at least 15-fold, at least 20-fold, at least 50-fold or at least 100-fold higher affinity to said peptide (tag) than to said corresponding non-mutated peptide of said human protein FGFR2,
      and wherein said inducible gene expression system is an antigen-activated inducible gene expression system, and said antigen-activated inducible promoter is an antigen-activated promoter capable of driving expression of said tagged polypeptide when said first immune cell is activated by said antigen.

In a preferred embodiment of the invention, the immune cell is a TIL comprising
i) a nucleic acid sequence (comprising) encoding an anti-tag CAR comprising
   a) an antigen binding domain specific for a tag of a tagged polypeptide, wherein said tag comprises SEQ ID NO:3 (P2M2)
   b) a transmembrane domain
   c) an intracellular signaling domain, and
ii) a nucleic acid sequence (comprising) encoding said tagged polypeptide,
   and wherein said polypeptide specifically binds to a tumor associated antigen such as CD20 or CD33,
   and wherein said antigen binding domain specific for said tag comprises SEQ ID NO:5 (anti-P2M2),
   wherein said immune cell additionally comprises an inducible gene expression system comprising
      a) said inducible gene expression system comprising
         I) a first nucleic acid sequence comprising an inducible promoter operably linked to a second nucleic acid sequence that is said nucleic acid sequence (comprising) encoding said tagged polypeptide
         II) a third nucleic acid sequence that is said nucleic acid sequence (comprising) encoding said anti-tag CAR,
            and wherein said inducible gene expression system is an antigen-activated inducible gene expression system, and said antigen-activated inducible promoter is an antigen-activated promoter capable of driving expression of said tagged polypeptide when said immune cell is activated by said antigen.

The TCR of such a TIL would cause homing of the adapter-producing cell to the site of the tumor and restrict Adapter CAR function to this location.

In one embodiment of the invention the immune cells such as a TIL, a T cell or an NK cell expressing both the Adapter CAR and the adapter as disclosed herein, may be for use in treatment of a disease associated with a target cell of a subject suffering from said disease, the disease may be e.g. cancer and the target cell a cancerous cell. Immune cells, e.g. TILs, T cells or NK cells of a subject may be isolated. The subject may e.g. suffer from said cancer or may be a healthy subject. These cells are genetically modified in vitro to express the Adapter CAR and the adapter as disclosed herein. These engineered cells may be activated and expanded in vitro. In a cellular therapy these engineered cells are infused to a recipient in need thereof. These cells may be a pharmaceutical composition (said cell plus pharmaceutical acceptable carrier). The infused cells may be e.g. able to kill (or at least stop growth of) cancerous cells in the recipient. The recipient may be the same subject from which the cells was obtained (autologous cell therapy) or may be from another subject of the same species (allogeneic cell therapy). The immune cells, preferentially TILs, T cells or NK cells engineered to express the Adapter CAR and the adapter as disclosed herein may be administered either alone, or as a pharmaceutical composition in combination with diluents and/or with other components such as IL-2 or other cytokines or cell populations. Briefly, pharmaceutical compositions of the present invention may comprise a cell population of genetically modified cells as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

Preferentially, the compositions of the present invention are formulated for intravenous administration. The administration of cell compositions to the subject may be carried out in any convenient manner known in the art.

Pharmaceutical compositions of the present invention may be administered in a manner appropriate to the disease to be treated. Appropriate dosages may be determined by clinical trials. But the quantity and frequency of administration will also be determined and influenced by such factors as the condition of the patient, and the type and severity of the patient's disease. A pharmaceutical composition comprising the immune cells, preferentially TILs, T cells or NK cells as disclosed herein may be administered at a dosage of 10⁴ to 10⁹ cells/kg body weight, preferably 10⁵ to 10⁶ cells/kg body weight. The cell compositions may also be administered several times at these dosages. The compositions of cells may be injected e.g. directly into a tumor, lymph node, or site of infection.

The genetically engineered immune cells may be activated and expanded to therapeutic effective amounts using methods known in the art.

The immune cells of the invention may be used in combination with e.g. chemotherapy, radiation, immunosuppressive agents, antibodies or antibody therapies.

All definitions, characteristics and embodiments defined herein with regard to the first aspect of the invention as disclosed herein also apply mutatis mutandis in the context of the other aspects of the invention as disclosed herein.

### Definitions

Unless defined otherwise, technical and scientific terms used herein have the same meaning as commonly understood by one of ordinary skill in the art to which this invention belongs. As used herein the term "comprising" or "comprises" is used in reference to compositions, methods, and respective component(s) thereof, that are essential to the method or composition, yet open to the inclusion of unspecified elements, whether essential or not.

In general, a CAR may comprise an extracellular domain (extracellular part) comprising the antigen binding domain, a transmembrane domain and a cytoplasmic signaling domain (intracellular signaling domain). The extracellular domain may be linked to the transmembrane domain by a linker (or spacer or hinge region). The extracellular domain may also comprise a signal peptide.

A "signal peptide" refers to a peptide sequence that directs the transport and localization of the protein within a cell, e.g. to a certain cell organelle (such as the endoplasmic reticulum) and/or the cell surface.

Generally, an "antigen binding domain" refers to the region of the CAR that specifically binds to an antigen, e.g. to a tumor associated antigen (TAA) or tumor specific antigen (TSA) or a tag of a tagged polypeptide, if the CAR is an Adapter CAR. The CARs may comprise one or more antigen binding domains (e.g. a tandem CAR). Generally, the targeting regions on the CAR are extracellular. The antigen binding domain may comprise an antibody or an antigen binding fragment thereof. The antigen binding domain may comprise, for example, full length heavy chain, Fab fragments, single chain Fv (scFv) fragments, divalent single chain antibodies, nanobodies or diabodies. Any molecule that binds specifically to a given antigen such as affibodies or ligand binding domains from naturally occurring receptors may be used as an antigen binding domain. Often the antigen binding domain is a scFv. Normally, in a scFv the variable regions of an immunoglobulin heavy chain and light chain are fused by a flexible linker to form a scFv. Such a linker may be for example the "(G₄/S)₃-linker".

In some instances, it is beneficial for the antigen binding domain to be derived from the same species in which the CAR will be used in. For example, when it is planned to use it therapeutically in humans, it may be beneficial for the antigen binding domain of the CAR to comprise a human or humanized antibody or antigen binding fragment thereof. Human or humanized antibodies or antigen binding fragments thereof can be made by a variety of methods well known in the art.

"Spacer" or "hinge" as used herein refers to the hydrophilic region which is between the antigen binding domain and the transmembrane domain. The CARs may comprise an extracellular spacer domain but is it also possible to leave out such a spacer. The spacer may include e.g. Fc fragments of antibodies or fragments thereof, hinge regions of antibodies or fragments thereof, CH2 or CH3 regions of antibodies, accessory proteins, artificial spacer sequences or combinations thereof. A prominent example of a spacer is the CD8alpha hinge.

The transmembrane domain of the CAR may be derived from any desired natural or synthetic source for such domain. When the source is natural the domain may be derived from any membrane-bound or transmembrane protein. The transmembrane domain may be derived for example from CD8alpha or CD28. When the key signaling and antigen recognition modules (domains) are on two (or even more) polypeptides then the CAR may have two (or more) transmembrane domains. Splitting key signaling and antigen recognition modules enable for a small molecule-dependent, titratable and reversible control over CAR cell expression (e.g. WO2014127261A1) due to small molecule-dependent heterodimerizing domains in each polypeptide of the CAR.

The cytoplasmic signaling domain (the intracellular signaling domain or the activating endodomain) of the CAR is responsible for activation of at least one of the normal effector functions of the immune cell in which the CAR is expressed, if the respective CAR is an activating CAR (normally, a CAR as described herein refers to an activating CAR, otherwise it is indicated explicitly as an inhibitory CAR (iCAR)). "Effector function" means a specialized function of a cell, e.g. in a T cell an effector function may be cytolytic activity or helper activity including the secretion of cytokines. The intracellular signaling domain refers to the part of a protein which transduces the effector function signal and directs the cell expressing the CAR to perform a specialized function. The intracellular signaling domain may include any complete, mutated or truncated part of the intracellular signaling domain of a given protein sufficient to transduce a signal which initiates or blocks immune cell effector functions.

Prominent examples of intracellular signaling domains for use in the CARs include the cytoplasmic signaling sequences of the T cell receptor (TCR) and co-receptors that initiate signal transduction following antigen receptor engagement.

Generally, T cell activation can be mediated by two distinct classes of cytoplasmic signaling sequences, firstly those that initiate antigen-dependent primary activation through the TCR (primary cytoplasmic signaling sequences, primary cytoplasmic signaling domain) and secondly those that act in an antigen-independent manner to provide a secondary or co-stimulatory signal (secondary cytoplasmic signaling sequences, co-stimulatory signaling domain). Therefore, an intracellular signaling domain of a CAR may comprise one or more primary cytoplasmic signaling domains and/or one or more secondary cytoplasmic signaling domains.

Primary cytoplasmic signaling domains that act in a stimulatory manner may contain ITAMs (immunoreceptor tyrosine-based activation motifs).

Examples of ITAM containing primary cytoplasmic signaling domains often used in CARs are that those derived from TCRζ (CD3ζ), FcRgamma, FcRbeta, CD3gamma, CD3delta, CD3epsilon, CD5, CD22, CD79a, CD79b, and CD66d. Most prominent is sequence derived from CD3ζ.

The cytoplasmic domain of the CAR may be designed to comprise the CD3ζ signaling domain by itself or combined with any other desired cytoplasmic domain(s). The cytoplasmic domain of the CAR can comprise a CD3ζ chain portion and a co-stimulatory signaling region (domain). The co-stimulatory signaling region refers to a part of the CAR comprising the intracellular domain of a co-stimulatory molecule. A co-stimulatory molecule is a cell surface molecule other than an antigen receptor or their ligands that is required for an efficient response of lymphocytes to an antigen. Examples for a co-stimulatory molecule are CD27, CD28, 4-1BB (CD137), OX40, CD30, CD40, ICOS, lymphocyte function-associated antigen- 1 (LFA-1), CD2, CD7, LIGHT, NKG2C, B7-H3.

The cytoplasmic signaling sequences within the cytoplasmic signaling part of the CAR may be linked to each other with or without a linker in a random or specified order. A short oligo- or polypeptide linker, which is preferably between 2 and 10 amino acids in length, may form the linkage. A prominent linker is the glycine-serine doublet.

As an example, the cytoplasmic domain may comprise the signaling domain of CD3ζ and the signaling domain of CD28. In another example the cytoplasmic domain may comprise the signaling domain of CD3ζ and the signaling domain of CD137. In a further example, the cytoplasmic domain may comprise the signaling domain of CD3ζ, the signaling domain of CD28, and the signaling domain of CD137.

As aforementioned either the extracellular part or the transmembrane domain or the cytoplasmic domain of a CAR may also comprise a heterodimerizing domain for the aim of splitting key signaling and antigen recognition modules of the CAR.

The CAR may be further modified to include on the level of the nucleic acid encoding the CAR one or more operative elements to eliminate CAR expressing immune cells by virtue of a suicide switch. The suicide switch can include, for example, an apoptosis inducing signaling cascade or a drug that induces cell death. In one embodiment, the nucleic acid expressing and encoding the CAR can be further modified to express an enzyme such thymidine kinase (TK) or cytosine deaminase (CD). The CAR may also be part of a gene expression system that allows controlled expression of the CAR in the immune cell. Such a gene expression system may be an inducible gene expression system and wherein when an induction agent is administered to a cell being transduced with said inducible gene expression system, the gene expression system is induced and said CAR is expressed on the surface of said transduced cell.

In some variants, the endodomain of a CAR may contain a primary cytoplasmic signaling domains or a co-stimulatory region, but not both.

A specific variant of a CAR is the Adapter CAR (or anti-tag CAR), that uses a bridging molecule, i.e. an adapter (or tagged polypeptide) for binding of the CAR to the target molecule, e.g. the antigen expressed on a target cell.

The antigen binding domain of an Adapter CAR binds a tag or hapten that is coupled to a polypeptide ("haptenylated" or "tagged" polypeptide), wherein the polypeptide e.g. may bind to a disease-associated antigen such as a tumor associated antigen (TAA) or tumor specific antigen that may be expressed on the surface of a cancer cell.

Such an Adapter CAR (AdCAR) may be referred to also as "anti-tag CAR" or "universal CAR". An anti-tag CAR has been disclosed first in US9233125B2.

The CAR part of the present invention is an anti-tag CAR comprising an antigen binding domain specific for a tag of a tagged polypeptide.

The anti-tag CARs of the present invention may be designed to comprise any portion or part of the above-mentioned domains of a CAR as described above and herein in any order and/or combination resulting in a functional CAR, i.e. a CAR that mediates an immune effector response of the immune effector cell that expresses the CAR as disclosed herein, when the anti-tag CAR binds to the tagged polypeptide as disclosed herein.

The term "tagged polypeptide" as used herein refers to a polypeptide that has bound thereto directly or indirectly at least one additional component, i.e. the tag. The tagged polypeptide as used herein may be able to bind an antigen expressed on a target cell. The polypeptide may be an antibody or antigen binding fragment thereof that binds to an antigen expressed on the surface of a target cell such as a tumor associated antigen on a cancer cell. The polypeptide of the tagged polypeptide alternatively may a cytokine or a growth factor or another soluble polypeptide that is capable of binding to an antigen of a target cell.

The terms "adapter" or "adapter molecule" or "tagged polypeptide" as used herein may be used interchangeably.

Often, the tag of an anti-tag CAR may be e.g. a hapten and the hapten may be bound by the antigen binding domain of the polypeptide, e.g. a CAR, comprising an antigen binding domain specific for the tag.

Haptens such as e.g. FITC, biotin, PE, streptavidin or dextran are small molecules that elicit an immune response only when attached to a large carrier such as a protein; the carrier may be one that also does not elicit an immune response by itself.

Whereas the tag part of the anti-tag CAR/tagged polypeptide system of the present invention is a peptide sequence e.g. chemically or recombinantly coupled to the polypeptide part of the tagged polypeptide, and wherein said peptide sequence (peptide) bears a characteristics as disclosed herein: said tag of said tagged polypeptide is a peptide of the human protein FGFR2, and wherein the amino acid sequence of said peptide may bear at least one mutation as compared to the corresponding non-mutated peptide (Wild type) of said human protein FGFR2, and wherein said polypeptide may specifically bind to an antigen, and wherein said antigen binding domain specific for said tag may bind with at least 10-fold higher affinity to said peptide (tag) than to said corresponding non-mutated peptide of said human protein FGFR2.

Said tag of said tagged polypeptide may comprise between 8 to 30 amino acids, between 8 to 20 amino acids, between 8 to 15 amino acids, between 9 to 11 amino acids or may have about 10 amino acids or may have 10 amino acids.

Said at least one mutation may be a mutation that is commonly mutated (or prevalent) in said human FGFR2 protein in cancer.

The term "wherein the amino acid sequence of said peptide bears at least one mutation as compared to the corresponding non-mutated peptide (Wild type) of said human protein FGFR2" as used herein refers to the selection of a peptide sequence of a domain of the human protein FGFR2 and introducing said at least one mutation into this peptide sequence.

Fibroblast growth factor receptor 2 (FGFR2) also known as CD332 is a protein that in humans is encoded by the FGFR2 gene residing on chromosome 10. FGFR2 is a receptor for fibroblast growth factor. Its complete amino acid sequence is displayed in Uniprot P21802.

The unchanged peptide sequence (i.e. the corresponding peptide sequence) matches with the amino acid sequence within the human protein FGFR2. For purpose of illustration only, the human protein may be FGFR2 and comprises 821 amino acids (Uniprot P21802), the peptide that bears at least one mutation (in this example 3 mutations) may then be SEQ ID NO:3 (having the mutations D247Y, S252W and P253R within the peptide that is aa 247-256 of the sequence of Uniprot P21802), and then the corresponding non-mutated peptide may be SEQ ID NO:1 (that is aa 247-256 of the sequence of Uniprot P21802).

The term "antibody" as used herein is used in the broadest sense to cover the various forms of antibody structures including but not being limited to monoclonal and polyclonal antibodies (including full length antibodies), multispecific antibodies (e.g. bispecific antibodies), antibody fragments, i.e. antigen binding fragments of an antibody, immunoadhesins and antibody-immunoadhesin chimeras, that specifically recognize (i.e. bind) an antigen. "Antigen binding fragments" comprise a portion of a full-length antibody, preferably the variable domain thereof, or at least the antigen binding site thereof ("an antigen binding fragment of an antibody"). Examples of antigen binding fragments include Fab (fragment antigen binding), scFv (single chain fragment variable), single domain antibodies (nanobodies), diabodies, dsFv, Fab', diabodies, single-chain antibody molecules, and multispecific antibodies formed from antibody fragments. The antibody or antibody fragment may be human, fully human, humanized, human engineered, non-human, and/or chimeric. The non-human antibody or antibody fragment may be humanized to reduce immunogenicity to humans, while retaining the specificity and affinity of the parental non-human antibody. Chimeric antibodies may refer to antibodies created through the joining of two or more antibody genes which originally encoded for separate antibodies.

The terms "having specificity for", "specifically binds" or "specific for" with respect to an antigen-binding domain of an antibody, of a fragment thereof or of a CAR refer to an antigen-binding domain which recognizes and binds to a specific antigen, but does not substantially recognize or bind other molecules in a sample. An antigen-binding domain that binds specifically to an antigen from one species may bind also to that antigen from another species. This cross-species reactivity is not contrary to the definition of that antigen-binding domain is specific. An antigen-binding domain that specifically binds to an antigen may bind also to different allelic forms of the antigen (allelic variants, splice variants, isoforms etc.). This cross reactivity is not contrary to the definition of that antigen-binding domain is specific.

As used herein, the term "antigen" is intended to include substances that bind to or evoke the production of one or more antibodies and may comprise, but is not limited to, proteins, peptides, polypeptides, oligopeptides, lipids, carbohydrates such as dextran, haptens and combinations thereof, for example a glycosylated protein or a glycolipid. The term "antigen" as used herein refers to a molecular entity that may be expressed e.g. on the surface of a target cell such as a cancer cell and that can be recognized by means of the adaptive immune system including but not restricted to antibodies or TCRs, or engineered molecules including but not restricted to endogenous or transgenic TCRs, CARs, scFvs or multimers thereof, Fab-fragments or multimers thereof, antibodies or multimers thereof, single chain antibodies or multimers thereof, or any other molecule that can execute binding to a structure with high affinity. The antigen may also be a soluble antigen. The term "soluble antigen" as used herein refers to an antigen that is not immobilized on surfaces such as beads or cell membranes.

The terms "immune cell" or "immune effector cell" may be used interchangeably and refer to a cell that may be part of the immune system and executes a particular effector function such as alpha-beta T cells, NK cells, NKT cells, B cells, innate lymphoid cells (ILC), cytokine induced killer (CIK) cells, lymphokine activated killer (LAK) cells, gamma-delta T cells, regulatory T cells (Treg), monocytes or macrophages. Preferentially these immune cells are human immune cells. Preferred immune cells are cells with cytotoxic effector function such as alpha-beta T cells, NK cells, NKT cells, ILC, CIK cells, LAK cells or gamma-delta T cells. Most preferred immune effector cells may be T cells, NK cells and/or Tumor infiltrating lymphocytes (TILs). Tumor infiltrating lymphocytes (TILs) are T cells that have moved from the blood of a subject into a tumor. These TILs may be removed from a patient's tumor by methods well known in the art, e.g. enzymatic and mechanic tumor disruption followed by density centrifugation and/or cell marker specific enrichment. TILs may be genetically engineered as disclosed herein, and then given back to the patient. "Effector function" means a specialized function of a cell, e.g. in a T cell an effector function may be cytolytic activity or helper activity including the secretion of cytokines.

Immunotherapy is a medical term defined as the "treatment of disease by inducing, enhancing, or suppressing an immune response". Immunotherapies designed to elicit or amplify an immune response are classified as activation immunotherapies, while immunotherapies that reduce or suppress are classified as suppression immunotherapies. Cancer immunotherapy as an activating immunotherapy attempts to stimulate the immune system to reject and destroy tumors. Adoptive cell transfer uses cell-based, preferentially T cell-based, NK cell-based or TIL-based cytotoxic responses to attack cancer cells. T cells that have a natural or genetically engineered reactivity to a patient's cancer are generated *in-vitro* and then transferred back into the cancer patient. If an immune cells is genetically engineered to express a CAR, then the immunotherapy is referred to as "CAR cell immunotherapy" or in case of use of T cells only as "CAR T cell therapy" or "CAR T cell immunotherapy".

The term "treatment" as used herein means to reduce the frequency or severity of at least one sign or symptom of a disease.

The term "autologous" as used herein refers to any material derived from the same subject to who it is later re-introduced.

The term "allogeneic" as used herein refers to any material derived from a different subject of the same species as the subject to who the material is re-introduced.

The terms "therapeutically effective amount" or "therapeutically effective population" mean an amount of a cell population which provides a therapeutic benefit in a subject.

As used herein, the term "subject" refers to an animal. Preferentially, the subject is a mammal such as mouse, rat, cow, pig, goat, chicken dog, monkey or human. More preferentially, the subject is a human. The subject may be a subject suffering from a disease such as cancer (a patient) or from an autoimmune disease or from an allergic disease or from an infectious disease or from graft rejection.

The term "expression" as used herein is defined as the transcription and/or translation of a particular nucleotide sequence driven by its promoter in a cell.

The terms "engineered cell" and "genetically modified cell" as used herein can be used interchangeably. The terms mean containing and/or expressing a foreign gene or nucleic acid sequence which in turn modifies the genotype or phenotype of the cell or its progeny. Especially, the terms refer to the fact that cells, preferentially T cells can be manipulated by recombinant methods well known in the art to express stably or transiently peptides or proteins which are not expressed in these cells in the natural state. For example, T cells, preferentially human T cells are engineered to express an artificial construct such as a chimeric antigen receptor on their cell surface.

The term "cancer" is known medically as a malignant neoplasm. Cancer is a broad group of diseases involving unregulated cell growth and includes all kinds of leukemia. In cancer, cells (cancerous cells) divide and grow uncontrollably, forming malignant tumors, and invading nearby parts of the body. The cancer may also spread to more distant parts of the body through the lymphatic system or bloodstream. There are over 200 different known cancers that affect humans.

The terms "nucleic acid" or nucleic acid sequence" or "polynucleotide" as used interchangeably herein refer to polymers of nucleotides. Polynucleotides, which can be hydrolyzed into monomeric "nucleotides." The monomeric nucleotides can be hydrolyzed into nucleosides. As used herein, the term "polynucleotides" encompasses, but is not limited to, all nucleic acid sequences which are obtained by any means available in the art, including, without limitation, recombinant means, i.e., the cloning of nucleic acid sequences e.g. from a recombinant library or a cell genome, using ordinary cloning technology and PCR, and the like, and by synthetic means.

Peptides are short chains of between two and fifty amino acids, linked by peptide bonds. Chains of less than ten or fifteen amino acids may also be called oligopeptides.

A polypeptide is a longer, continuous, unbranched peptide chain of up to fifty amino acids or more. A polypeptide that contains more than fifty amino acids may also called a protein.

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

As used herein, the terms "promoter" or "regulatory sequence" mean a nucleic acid sequence which is required for transcription of a gene product operably linked to the promoter/regulatory sequence. In some instances, this sequence may be the core promoter sequence and in other instances, this sequence may also include an enhancer sequence and other regulatory elements which are required for transcription of the gene product. The promoter/regulatory sequence may, for example, be one which expresses the gene product in a tissue-specific manner.

The term "minimal promoter (PMIN)" as used herein refers to the smallest genetic element that is able to induce transcription of a gene located downstream of said minimal promoter. Eukaryotic promoters of protein-coding genes have one or more of three conserved sequences in this region (i.e. the TATA-box, initiator region, and downstream promoter element). A minimal promoter enables low basal leakiness in the absence of specific transcriptional activators and high expression when transcription activators are bound upstream of minimal promoter at their specific DNA binding sites. Alternative minimal promoters can be used, such as minimal TATA box promoter, minimal CMV promoter or minimal IL-2 promoter.

The minimal promoter may be engineered / modified by the introduction of binding sites for specific transcription factors (e.g. required for the drug-inducible system, but also for the antigen-activated inducible system the promoter may contain several repeats e.g. of the NFAT binding site).

A "constitutive" promoter is a nucleotide sequence which, when operably linked with a polynucleotide which encodes or specifies a gene product, causes the gene product to be produced in a cell under most or all physiological conditions of the cell.

An "inducible" promoter is a nucleotide sequence which, when operably linked with a polynucleotide which encodes or specifies a gene product, causes the gene product to be produced in a cell substantially only in the presence or absence of certain conditions such as, for example, when an inducer ( e.g. an induction signal, or an induction agent such as a drug, metal ions, alcohol, oxygen, etc.) is present in the cell. The inducer may be e.g. the activation of the intracellular signaling domain of a CAR.

Constitutive promotors that are operatively linked to a transgene may be for example EF-l alpha promoter or any other constitutive promoter that drives constitutive expression in immune cells (such as MSCV, PGK-l, UBC, CMV, CAGG, SV40 or pan-hematopoietic promoter, such as vav).

The inducible promoter that is operably linked to the polynucleotide encoding e.g. the adapter, i.e. the tagged polypeptide as disclosed herein may be any promoter the activation of which is responsive to a transcriptional factor that is increased when immune cells are specifically activated or localized to a given microenvironment (could be e.g. a tumor microenvironment), such as an SP1, BATF, AP-1, IRF4, RUNX, NFAT, NF-κB, STATS or STATS sensing promoter and a minimal promoter operably linked to an inducible enhancer as described e.g. in WO2019199689A1. The inducible promoter further may comprise a minimal promoter operably linked to an effector. NFAT -inducible promoter can be exchanged with any inducible promoter that specifically binds specific transcriptional factors. Without wishing to be bound by theory, if NFAT responsive element is present, it needs a signal from TCR/CAR or other immune receptors that induces NFAT signaling.

The inducible promoter may comprise a minimal promoter (PMIN). Alternative minimal promoters can be used, such as minimal TATA box promoter, minimal CMV promoter or minimal IL-2 promoter.

The term "inducible (gene) expression system" refers to the expression of an exogenous polypeptide (a transgene, herein normally the adapter, i.e. the tagged polypeptide as disclosed herein in an immune cell. The inducible (gene) expression system may be an "antigen-activated inducible gene expression system", i.e. the inducible expression system may be activated in a cell having said inducible gene expression system, when an antigen/ligand is bound directly or upon MHC presentation by a receptor of the cell such as a CAR or a TCR. Said binding of the antigen/ligand to the receptor may induce a signal cascade within the cell that subsequently may lead to the induction of the expression of the introduced transgene. Said antigen/ligand that may induce a signal cascade within the cell, when bound to the cognate receptor of the cell may also be referred to as "inducing signal".

Alternatively, the inducible gene expression system may be a drug-inducible gene expression system, i.e. the inducible gene expression system may be activated in a cell having said inducible gene expression system, when a drug, e.g. a synthetic drug such as 4-hydroxytamoxifen or endoxifen may be introduced to the cell. Said drug in the cell may bind to a synthetic transcription factor and subsequently may lead to the induction of the expression of the transgene, herein the adapter.

Said drug may also be referred to as "inducing agent".

Both kinds of inducible gene expression systems may be used in a one cell and/or two cell system as disclosed herein.

In the presence of an induction signal the inducible expression system drives expression of the exogenous polypeptide. In an induced system, withdrawal of the induction signal may reduce and/or halt expression of the exogenous polypeptide. Upon re-introduction of the induction signal the system can then be re-induced and restart the expression of the exogenous polypeptide. The inducible (gene) expression system may be inducible by an induction signal, e.g. by the activation of the intracellular signaling domain of a CAR or a TCR by binding of an antigen/ligand as disclosed herein ("activation"-induced) or by an induction agent, such as a (synthetic) drug as disclosed herein ("drug-induced").

The term "epitope" means the part of an antigen, e.g. the tag of the tagged polypeptide, that may be recognized and specifically bound by antibodies or antigen bindings fragments thereof (antigen binding domains).

The term "tumor associated antigen" (TAA) as used herein refers to an antigenic substance produced in tumor cells. Tumor associated antigens are useful tumor or cancer markers in identifying tumor/cancer cells with diagnostic tests and are potential candidates for use in cancer therapy. Preferentially, the TAA may be expressed on the cell surface of the tumor/cancer cell, so that it may be recognized by the antigen binding receptor as disclosed herein.

The term "target cell" as used herein refers to cell which expresses an antigen on its cell surface that should be recognized (bound) by the antigen binding domain of the tagged polypeptide as disclosed herein. Said target cell may be e.g. a cancerous cell or a cell associated with an autoimmune disease or a cell associated with an infectious disease.

The term "isolated" is used herein to indicate that e.g. the polypeptide, nucleic acid sequence or host cell exist in a physical milieu distinct from that in which it occurs in nature. For example, the isolated polypeptide may be substantially isolated (for example enriched or purified) with respect to the complex cellular milieu in which it naturally occurs, such as in a crude extract. A "kit" may comprise a container with components within the container. Such containers may be e.g. boxes, bottles, vials, tubes, bags, pouches, blister packs, or other suitable container forms known in the art. Such containers may be made of plastic, glass, laminated paper, metal foil, or other materials suitable for holding components therein. The kit further may comprise written directions for using the components of the kits.

### Examples

The following examples are intended for a more detailed explanation of the invention but without restricting the invention to these examples.

### Example 1: FGFR2-derived epitope tags and functionality of Adapter CAR T cells directed against FGFR2 epitope tags (CD20+ target cells)

In order to generate CARs specific for a peptide derived from a mutated from of FGFR2 commonly found in cancer (Table 1), a human scFv library was subtractively screened for binders against the P2M2 FGFR1/2 peptide sequence (carrying 3 amino acid mutations away from the native FGFR1/2 peptide, SEQ ID NO:3), while excluding scFvs that recognized the native FGFR1/2 WT, SEQ ID NO: 1 sequence.

**Table 1: Mutations in interdomain region B of FGFR2 commonly found in cancer**

| **MUTATION INTERDOMAIN REGION B FGFR2** | **DISEASE** | **REFERENCE** |
|---|---|---|
| D247Y | Lung squamous cell carcinoma | Tchaicha JH et al., Cancer Res 2014;74:4676-84. |
| S252W | Endometrial cancer, Gallbladder cancer | Dutt A et al., Proc Natl Acad Sci U S A 2008;105:8713-7. |
| | | Byron SA, et al. PLoS ONE 2012;7:e30801. |
| | | Dulak AM, et al. Nat Genet 2013;45:478-86. |
| P253R | Endometrial cancer, Oral squamous cell carcinoma | Dutt A et al., Proc Natl Acad Sci U S A 2008; 105:8713-7. |
| | | Byron SA, et al. PLoS ONE 2012;7:e30801. |
| | | Liao RG, et al. Cancer Res 2013;73:5195-205. |

Fab-based adapters were generated against CD20 to determine the efficacy of the Adapter CAR, with the specific FGFR2 P2M2 epitope tag fused to the C-terminus of the heavy chain constant region (Figure 1A; mutations away from the WT FGFR2 peptide are shown in red). In order to demonstrate the lack of reactivity of the scFv against the native FGFR1/2 protein peptide, initial in vitro efficacy studies for the Adapter CAR against a cell line that expresses high levels of FGFR1 (Figure 1B), containing the native FGFR1/2 WT peptide sequence, were conducted. Adapter CAR-T cells were cocultured with FGFR1+/CD20+ Raji cells, a non-Hodgkin's lymphoma B cell line, in the presence or absence of 500ng/mL anti-CD20 Fab adapter carrying the indicated FGFR2 epitope tags (n=2 independent donors). Notably, only the specific FGFR2 P2M2 epitope tag led to Adapter CAR-T mediated cytotoxicity of the Raji cells (Figure 1C). The anti-CD20 (a-CD20) adapter with the WT FGFR2 peptide tag led to no appreciable cytotoxicity of the Raji cells relative to the control coculture of untransduced (UTD) T cells, nor did adapter with the P4M1 peptide tag, which carries one of three mutations away from the WT FGFR2 peptide sequence (S6W). The pattern of cytokine production (IL2) from CAR-T cells was similar, with only the P2M2 a-CD20 adapter increasing cytokine production from Adapter CAR-T cells above that seen with UTD cocultures with Raji cells (Figure 1D). Using both, a cell line expressing high levels of FGFR1 as well as the use of an adapter carrying the WT FGFR2 epitope tag, Adapter CAR T cells were demonstrated to be highly specific for the particular P2M2 peptide sequence, as well as unlikely to be cross-reactive with native FGFR2 proteins. To demonstrate the dose-responsive effect of the adapter, Adapter CAR-T cells were cocultured with CD20+ Raji cells, at different concentrations of a-CD20 P2M2. As positive control direct anti-CD20 CAR (scFv clone Leu16) T cells were used to assess the relative efficacy of the Adapter CAR T cells (n=3 independent donors). The Adapter CAR T cells demonstrated a dose-dependent response with the a-CD20 P2M2 adapter, with all doses (5-500 ng/mL) inducing Raji cytotoxicity (Figure 1D), CAR-T activation as measured by CD69 expression (Figure 1E) and TNFα production from Adapter CAR-T cells (Figure 1F). These responses were broadly equivalent to that observed with a direct anti-CD20 CAR (Figure 1D-F).

Next the efficacy of the anti-P2M2 (a-P2M2) Adapter CAR was assessed by in vivo studies of tumor clearance, again using CD20+ Raji NHL cells engineered to express firefly luciferase to monitor tumor expansion. Briefly, 5×10⁵ Raji cells were injected intravenously into NSG mice (NOD.Cg-Prkdc^{scid} Il2rg^{tm1Wjl}/SzJ) at Day -5 prior to CAR-T injection. On Day 0 of CAR-T engraftment, 6×10⁶ CAR-T cells (2×10⁷ total T cells) were injected. From Day 0 - Day 19 post-CAR-T engraftment, 50µg of anti-CD20 Fab adapter with the P2M2 tag (P2M2 a-CD20) was injected intraperitoneally each day, at which point the adapter treatment was stopped to determine the durability of the tumor clearance from the mice. Bioluminescent imaging was carried out at the indicated time points to monitor tumor expansion, and tumor luminescence is shown for each mouse in Figure 1G. Adapter CAR T cells, in the presence of P2M2 a-CD20 Adapter, lead to a durable tumor clearance response in 4 of 5 mice, as the tumor did not expand in the 16 day period following adapter treatment. The anti-tumor response with a-P2M2 Adapter CAR T cells was similar to that seen in using a direct anti-CD20 CAR T cells (Leu16 scFv, Figure 1G), where tumor clearance was observed in 5 of 5 mice. Notably, native mouse FGFR1/2 carries the same peptide sequence found in human FGFR1/2, and no observable toxicity occurred as a result of the Adapter CAR T cell treatment (data not shown), demonstrating a lack of cross-reactivity of the Adapter CAR with the native FGFR2 sequence.

### Example 2: Functionality of Adapter CAR T cells directed against FGFR2 P2M2 epitope tag (CD33+ target cells)

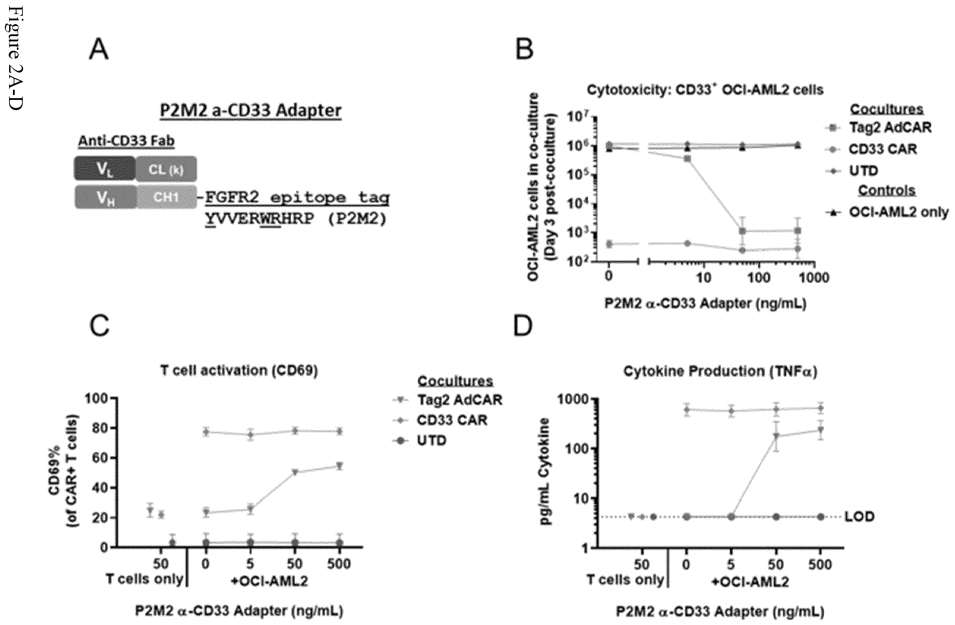

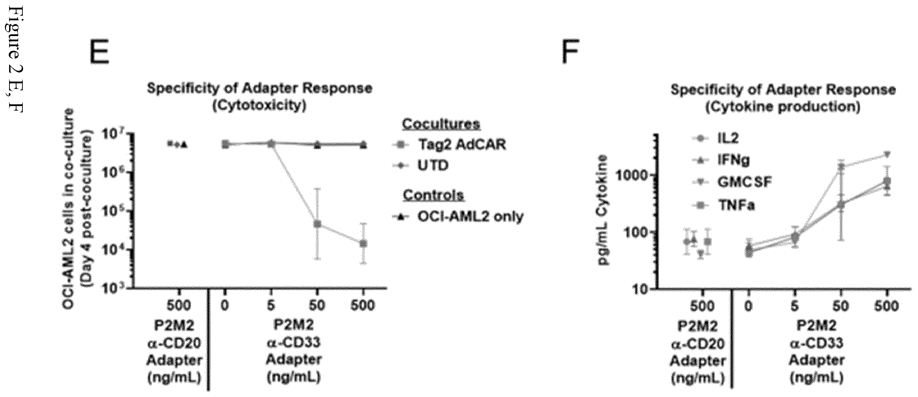

As the Adapter CAR should be retargetable to different antigens, the prior work on CD20+ Raji NHL cells was extended to CD33⁺ OCI-AML2 acute myelogenous leukemia (AML) tumor cells. Fab-based adapters were generated against CD33 to determine the efficacy of the Adapter CAR, with the specific FGFR2 P2M2 epitope tag fused to the C-terminus of the heavy chain constant region (anti-CD33 P2M2, Figure 2A; mutations away from the WT FGFR2 peptide are shown in red). Adapter CAR-T cells were cocultured with CD33+ OCI-AML2, at different concentrations of anti-CD33 P2M2 (n=3 independent donors). A positive control direct anti-CD33 CAR (scFv clone My96) was used to assess the relative efficacy of the Adapter CAR. The Adapter CAR demonstrated a dose-dependent response with the anti-CD33 P2M2 adapter, with both 500ng/mL and 50ng/mL inducing OCI-AML2 cytotoxicity (Figure 2B), CAR-T activation as measured by CD69 expression (Figure 2C) and TNFα production from Adapter CAR-T cells (Figure 2D). While the cytotoxic effect on OCI-AML2 cells from Adapter CAR-T cells was broadly equivalent to that produced by anti-CD33 direct CAR-T cells, the T cell activation and cytokine production induced in the presence of adapter was slightly less (Figure 2C,D). To demonstrate the specificity of the adapter response, a separate experiment was carried out where Adapter CAR-T cells were cocultured with CD33+ OCI-AML2, at different concentrations of anti-CD33 P2M2 (5-500 ng/mL) as well as 500 ng/mL anti-CD20 P2M2 (n = 2 independent donors). No cytotoxic response was observed in the presence of the anti-CD20 P2M2 adapter, while the anti-CD33 P2M2 adapter demonstrated equivalent cytoxicity as previously (Figure 2E). Correspondingly, only the anti-CD33 P2M2 Adapter, and not the anti-CD20 P2M2 Adapter induced production of a range of cytokines from Adapter CAR-T cells in coculture with CD33+ OCI-AML2 cells. Therefore, the anti-P2M2 Adapter CAR is retargetable against multiple classes of antigens, which is highly dependent on the specificity of the P2M2-tagged Adapter.

### Example 3: In situ production of adapters using FGFR2 P2M2 epitope tag

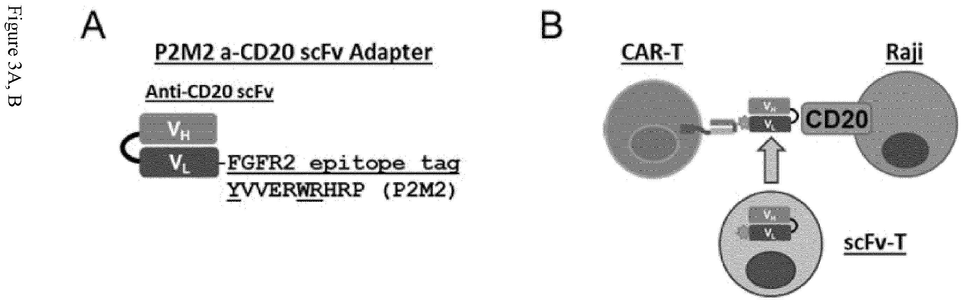

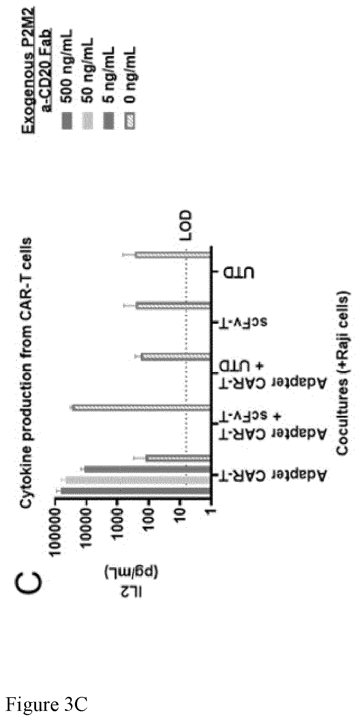

An advantage of the use of a recombinant peptide (e.g., P2M2) as the tag recognized by the Adapter CAR is the ability to produce the adapter in situ by a separate cell product, or adapter-producing cells. This would be advantageous if tumor-infiltrating lymphocyte T cells (TILs) were used, as the TCR would cause homing of the adapter-producing TILs to the site of the tumor and restrict Adapter CAR function to this location. As a proof of principle of this concept, an anti-CD20 scFv (scFv clone: Leu16) was engineered with a P2M2 tag as an adapter (Figure 3A). The P2M2 anti-CD20 scFv adapter was constitutively expressed in T cells (scFv-T), and triple cocultures were performed with anti-P2M2 Adapter CAR-T, scFv-T, and CD20+ Raji cells (Figure 3B). As a positive control, dual cocultures were performed with anti-P2M2 Adapter CAR-T and Raji cells, with the addition of exogenous P2M2 anti-CD20 Fab adapter. Importantly, the production of P2M2 anti-CD20 scFv adapter by the scFv-T cells induced IL2 cytokine production from the Adapter CAR-T cells (Figure 3C). This IL2 cytokine production was similar to that observed in Adapter CAR-T / Raji cocultures where 5-50 ng/mL of exogenous P2M2 anti-CD20 Fab adapter was added. These results indicate that it is possible to use a separate cell product to produce the adapter(s) in situ, and that this adapter is functional in activating the Adapter CAR-T cells when the cognate tumor antigen is present.

## Claims

1. A composition comprising
i) an anti-tag CAR comprising
a) an antigen binding domain specific for a tag of a tagged polypeptide
b) a transmembrane domain
c) an intracellular signaling domain, and
ii) said tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2,
and wherein said polypeptide specifically binds to an antigen,
and wherein said antigen binding domain specific for said tag binds with at least 10-fold higher affinity to said peptide than to said corresponding non-mutated peptide of said human protein FGFR2.

2. Said composition according to claim 1, wherein said at least one mutation is a mutation that is commonly mutated in said human protein FGFR2 in cancer.

3. Said composition according to claims 1 to 2, wherein said peptide of said human protein FGFR2 comprises SEQ ID NO:3 and wherein said antigen binding domain specific for a tag comprises SEQ ID NO:5, or wherein said peptide of said human protein FGRF2 comprises SEQ ID No:2 and wherein said antigen binding domain specific for a tag comprises SEQ ID NO:4.

4. An immune cell comprising
i) a nucleic acid sequence encoding an anti-tag CAR comprising
a) an antigen binding domain specific for a tag of a tagged polypeptide
b) a transmembrane domain
c) an intracellular signaling domain, and
ii) a nucleic acid sequence encoding said tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2, and wherein said polypeptide specifically binds to an antigen, and wherein said antigen binding domain specific for said tag binds with at least 10-fold higher affinity to said peptide than to said corresponding non-mutated peptide of said human protein FGFR2.

5. An immune cell according to claim 4, wherein said immune cell additionally comprises an inducible gene expression system comprising
I) a first nucleic acid sequence encoding an inducible promoter operably linked to a second nucleic acid sequence that is said nucleic acid sequence encoding said tagged polypeptide
II) a third nucleic acid sequence that is said nucleic acid sequence encoding an anti-tag CAR, and wherein said inducible gene expression system is an antigen-activated inducible gene expression system and said antigen-activated inducible promoter is an antigen-activated promoter capable of driving expression of said tagged polypeptide when said immune cell is activated by said antigen.

6. Said immune cell according to claims 4 or 5, wherein said peptide of said human protein FGRF2 comprises SEQ ID NO:3 and wherein said antigen binding domain specific for a tag comprises SEQ ID NO:5, or wherein said peptide of said human protein FGFR2 comprises SEQ ID No:2 and wherein said antigen binding domain specific for a tag comprises SEQ ID NO:4.

7. Said immune cell according to claims 4 to 6, wherein said immune cell is a tumor infiltrating lymphocyte (TIL).

8. A composition comprising
a) a first immune cell comprising an inducible gene expression system comprising a first nucleic acid sequence encoding an inducible promoter operably linked to a second nucleic acid sequence encoding a tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2, and wherein said polypeptide specifically binds to an antigen,
b) a second immune cell comprising a third nucleic acid sequence encoding an anti-tag CAR comprising
i) an antigen binding domain specific for a tag of a tagged polypeptide
ii) a transmembrane domain
iii) an intracellular signaling domain, and
and wherein said antigen binding domain specific for said tag binds with at least 10-fold higher affinity to said peptide than to said corresponding non-mutated peptide of said human protein FGFR2,
and wherein said inducible gene expression system is an antigen-activated inducible gene expression system and said antigen-activated inducible promoter is an antigen-activated promoter capable of driving expression of said tagged polypeptide when said first immune cell is activated by said antigen.

9. A composition comprising
a) a first immune cell comprising an inducible gene expression system comprising a first nucleic acid sequence comprising an inducible promoter operably linked to a second nucleic acid sequence encoding a tagged polypeptide, wherein said tag of said tagged polypeptide is a peptide of the human protein FGFR2, and wherein the amino acid sequence of said peptide bears at least one mutation as compared to the corresponding non-mutated peptide of said human protein FGFR2, and wherein said polypeptide specifically binds to an antigen,
b) a second immune cell comprising a third nucleic acid sequence encoding an anti-tag CAR comprising
i) an antigen binding domain specific for a tag of a tagged polypeptide
ii) a transmembrane domain
iii) an intracellular signaling domain,
and wherein said antigen binding domain specific for said tag binds with at least 10-fold higher affinity to said peptide than to said corresponding non-mutated peptide of said human protein FGFR2,
and wherein said inducible gene expression system is a drug-inducible expression system and said inducible promoter is a drug-inducible promoter, wherein said inducible gene expression system further comprises a nucleic acid encoding a synthetic transcription factor for said drug-inducible promoter, wherein when a drug is administered to a cell having said inducible gene expression system, the gene expression system is induced, and the tagged polypeptide is expressed.

10. The composition according to claim 9, wherein said synthetic transcription factor comprises a DNA binding domain and drug-binding domain and an activation domain, wherein said synthetic transcription factor is activated by binding to said drug.

11. The composition according to claims 8 to10, wherein said first immune cell is a TIL, and wherein said second immune cell is a T cell, NK cell or TIL.
